(19) Europäisches Patentamt
European Patent Office
Office européen des brevets

(11) Numéro de publication: **0 233 105**
**B1**

(12) # FASCICULE DE BREVET EUROPEEN

(45) Date de publication du fascicule du brevet: **09.01.91**

(21) Numéro de dépôt: **87400107.6**

(22) Date de dépôt: **16.01.87**

(51) Int. Cl.$^5$: **A 61 K 31/35,** C 07 D 311/30, C 07 H 15/26, C 07 H 17/04, C 07 H 17/07

(54) **Compositions thérapeutiques à base de dérivés de 3-alkoxyflavones et nouveaux dérivés de 3-alkoxyflavones.**

(30) Priorité: **17.01.86 FR 8600644**

(43) Date de publication de la demande:
**19.08.87 Bulletin 87/34**

(45) Mention de la délivrance du brevet:
**09.01.91 Bulletin 91/02**

(84) Etats contractants désignés:
**AT BE CH DE ES FR GB GR IT LI LU NL SE**

(56) Documents cités:
**EP-A-0 019 081**
**FR-A-2 187 320**
**FR-M- 3 541**
**US-A-3 632 477**
**US-A-3 661 890**
**US-A-3 867 541**

**PATENT ABSTRACTS OF JAPAN, vol. 7, no. 201 (C-184)1346r, 6 septembre 1983; & JP-A-58 99 414 (JIYUN OKUDA) 13-06-1983**

**Le dossier contient des informations techniques présentées postérieurement au dépôt de la demande et ne figurant pas dans le présent fascicule.**

(73) Titulaire: **INSTITUT PASTEUR**
**28, rue du Docteur Roux**
**F-75724 Paris Cédex 15 (FR)**

(72) Inventeur: **Vanden Berghe, Dirk A.R.**
**Heirweg 154**
**B-9270 Laarne (BE)**
Inventeur: **Vlietinck, Arnold J.**
**Boswachtersdreef 26**
**B-2180 Kalmthout (BE)**
Inventeur: **Haemers, Achiel**
**Voskenslaan 399**
**B-9000 Gand (BE)**

(74) Mandataire: **Polus, Camille et al**
**c/o Cabinet Lavoix 2, Place d'Estienne d'Orves**
**F-75441 Paris Cedex 09 (FR)**

**EP 0 233 105 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne des compositions thérapeutiques à base de dérivés de 3-alkoxyflavones, ayant notamment une activité antivirale.

Les dérivés de 3-alkoxyflavones sont des composés présentant la structure de base suivante

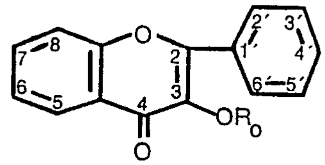

dans laquelle Ro est un groupe alkyle.

Des 3-méthoxyflavones à activité thérapeutique ont été décrites dans JP—A—58 99 414 et FR—17—3541.

Dans EP 0 019 081 on a décrit des dérivés de 3-alkoxyflavones présentant une activité antivirale. Les dérivés possèdent en position 5 un groupe hydroxy ou un groupe dérivé et en position 7 un groupe hydroxy ou alkoxy, les positions 6 et 8 étant non substituées. Ces composés sont décrits comme inhibant la reproduction de rhinovirus, d'entérovirus, de coxsackie-virus et de polio-virus.

Cependent certains de ces 3-alkoxyflavones présentent des rapports dose cytotoxique/dose active relativement faibles interdisant en pratique toute application thérapeutique. D'autres se sont mêmes avérées inactives.

On a maintenant trouvé que certaines classes particulières de 3-alkoxyflavones présentent une activité antivirale importante et avec des rapports dose cytotoxique/dose active permettant une application thérapeutique.

La présente invention a ainsi pour objet une composition thérapeutique comprenant à titre de principe actif un composé de formule

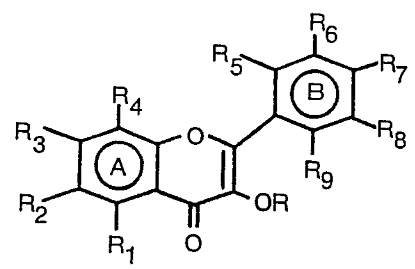

I

dans laquelle

R est un groupe alkyle en $C_1$—$C_4$ et

$R_1$, $R_2$, $R_3$, $R_4$ sont choisis parmi un atome d'hydrogène, un groupe hydroxy et un groupe alkoxy en $C_1$—$C_4$, trois de ces groupes étant autres que l'hydrogène,

$R_5$ et $R_9$ sont choisis parmi un atome d'hydrogène, un groupe hydroxy et un groupe alkoxy en $C_1$—$C_4$,

$R_6$ et $R_8$ sont des atomes d'hydrogène, et

$R_7$ est un groupe hydroxy, alcoxy en $C_1$—$C_4$ ou glycosyloxy;

Les composés utilises sont des produits d'origine naturelle ou dérivant de produits d'origine naturelle.

D'une manière générale les 3-alkoxyflavones utilisées dans la présente invention peuvent être obtenues par l'un des procédés suivants:

1) Alkylation de 3-hydroxyflavones selon le schéma suivant:

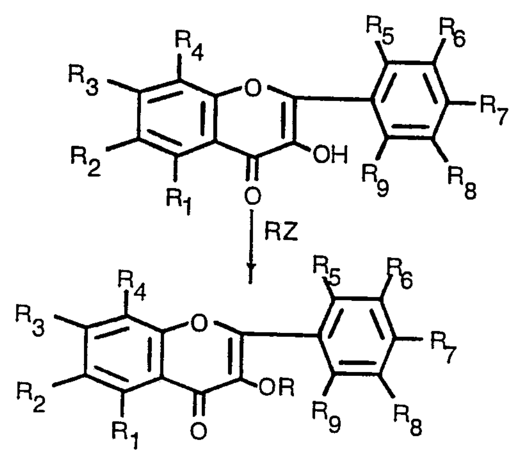


III

I

dans lequel z est un groupe nucléofuge.

Comme exemple d'agent de méthylation on peut citer par exemple le sulfate de méthyle, l'iodure de méthyle ou le diazométhane.

2) Synthése par la méthode de Allan Robinson (J. Chem. Soc. 125, 2192, 1924) par condensation de o-hydroxyarylcétones avec des anhydrides d'acides aromatiques selon le schéma suivant:

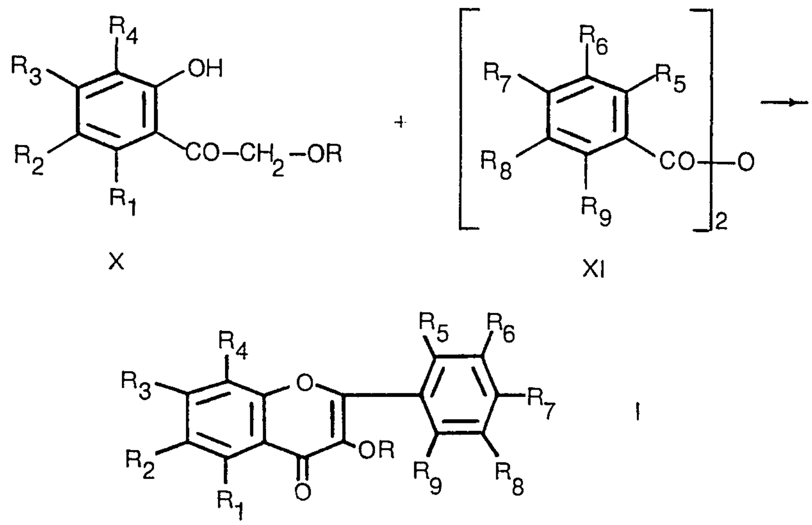

X XI

I

Dans cette méthode les groupes hydroxy éventuels peuvent être protégés si nécessaire et déprotéges après réaction.

En variante selon la même type de réaction on peut préparer une 3-hydroxyflavone et effectuer ensuite une alkylation comme décrit en 1.

3) Méthode d'oxydation de Algar-Flynn-Oyamada (J. Algar. J. P. Flynn, Proc. Roy Irish Acad. 423, 1, 1934, B. Oyamada J. Chem. Soc. Japan 55, 1256, 1934) consistant en l'oxydation de chalcones par du peroxyde d'hydrogène en milieu alcalin, suivie d'une réaction d'alkylation comme décrit en 1 selon le schéma suivant

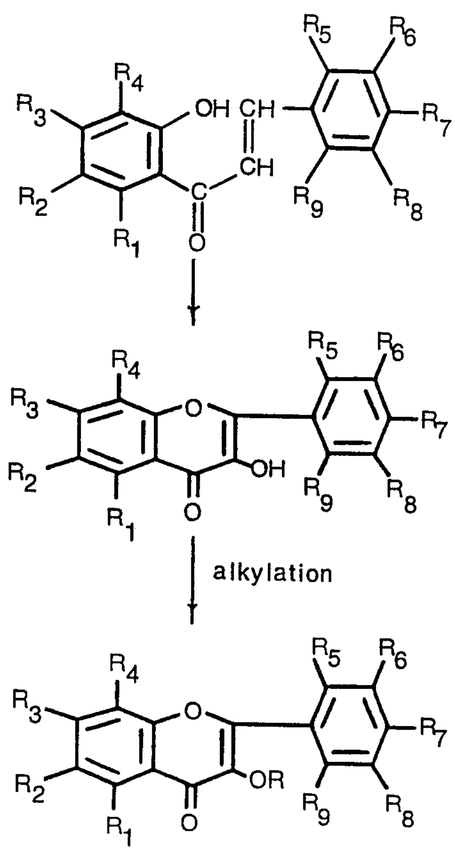


II

III

I

Dans cette méthode les groupes hydroxy éventuels peuvent être protégés et déprotégés à la fin.

4) Réduction nucléaire d'un ester, par exemple selon le schéma réactionnel suivant:

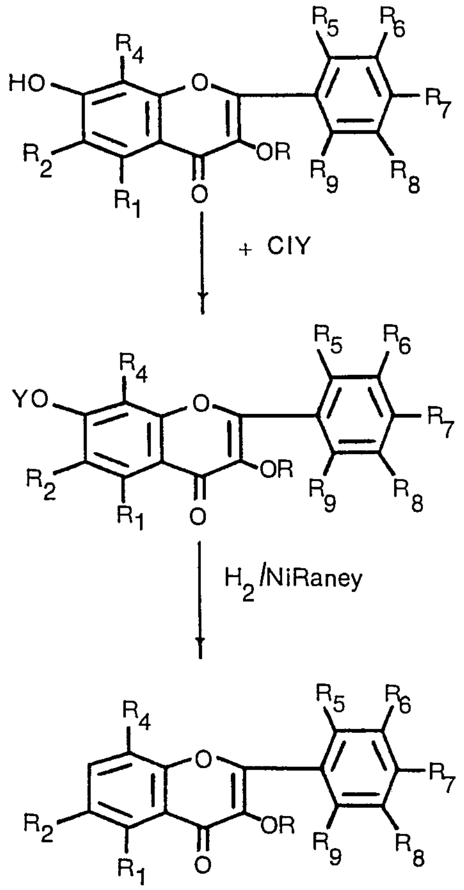


XIV

XV

XVI

Y étant par exemple un reste p. toluenesulfonyle.

5) Oxydation nucléaire, par exemple par du persulfate selon le schéma suivant:

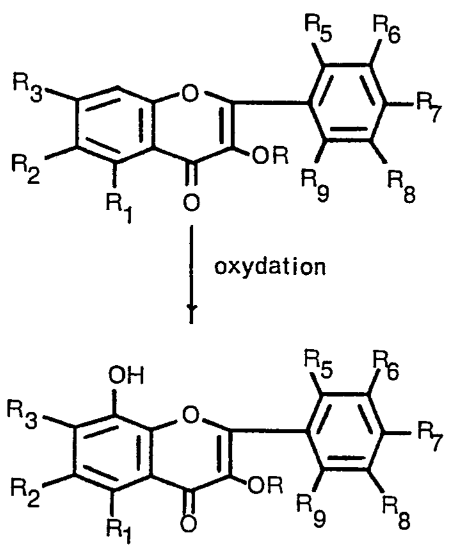


XVII

XVIII

Un certain nombre de 3-alkoxyflavones utilisables dans la présente invention sont présentes dans des plantes et peuvent être obtenues par extraction.

La méthode d'extraction est la suivante: On fait macérer les feuilles des plantes avec de l'éthanol à 80% sous agitation à température ambiante pendant 2 heures. On sépare les feuilles après macération par filtration et on percole le résidu de filtration avec le même solvant. On réunit le filtrat et le liquide de percolation et on concentre sous vide (à une température inférieure à 40°C) pour obtenir un extrait épais. L'extrait est soumis à un partage entre du méthanol à 90% et de l'hexane (ou un éther de pétrole 40—60°).

On sépare la phase méthanolique et on l'amène à siccité. Le résidu obtenu est soumis à un partage entre l'eau (pH 5) et l'acétate d'éthyle. On sépare la phase acétate d'éthyle et on l'amène à siccité. Le résidu obtenu est soumis à une chromatographie à contre courant, goutte à goutte, en utilisant comme éluant un mélange chloroforme/eau/méthanol (7:8:13) en mode descendant. On contrôle l'éffluent à 355 nm et par chromatographie en couche mince ou par chromatographie liquide haute pression et on test l'activité antivirale des fractions. Les fractions à activité antivirale sont soumises après évaporation à une chromatographie sur Sephadex® LH 20 en utilisant le méthanol comme éluant, et l'on contrôle l'éluat comme précédemment. On soumet les fractions actives à une chromatographie sur une colonne de gel de silice imprégné avec 5% d'eau et l'on développe en utilisant comme solvant un mélange hexane/acétate d'éthyle/acide acetique (5:3:1) selon la technique de la "colonne sèche". On obtient ainsi les 3-alkoxyflavones actives pures.

L'exemple suivant illustre la préparation des composés de formule I.

Exemple

Préparation de 4'-hydroxy-3-méthoxyflavone (Méthode d'Algar-Flynn-Oyamada)

a) Préparation de 2'-hydroxy-4-benzyloxychalcone

Une solution de 2-hydroxyacétophénone (25 mmole) et le benzyloxybenzaldéhyde (25 mmole) dans 25 ml d'éthanol est refroidie à la glace. On ajoute lentement une solution d'hydroxyde de potassium à 50% (25 ml) et on maintient le mélange sous agitation pendant 4 jours à température ambiante. On refroidit à nouveau à la glace la solution, et acidifie avec de l'acide chlorhydrique dilué. On sépare le précipité par filtration, lave à l'eau et recristallise dans l'éthanol (rendement 59%), F = 112—113°C.

On préparer de même d'autres chalcones.

b) Préparation de la 3-hydroxy-4'-benzyloxyflavone

On ajoute une solution à 10% d'hydroxyde de potassium dans de l'eau (15 ml) et une solution de péroxyde d'hydrogène (100 vol. 2 ml) à une solution à l'ébullition de la chalcone décrite en *a* (3 mmole) dans de l'éthanol (15 ml). Au bout de 15 minutes on verse la solution dans un mélange de glace et d'acide chlorhydrique et on sépare le précipité formé par filtration. On le lave à l'eau et le recristallise dans un mélange éthanol-acétone (rendement 42%) F = 176°C.

On peut préparer de même d'autres 3-hydroxyflavones.

c) Préparation de la 3-méthoxy-4'-benzyloxyflavone

On ajoute du carbonate de potassium (3 mmoles) et du sulfate de diméthyle (2 mmoles) à une solution de la 3-hydroxyflavone obtenue en b (1 mmole) dans de l'acétone. On chauffe le mélange au reflux pendant 12 heurs. On évapore le solvant. On ajoute de l'eau jusqu'à dissolution du précipité. On filtre le précipité et on recristallise dans l'éthanol (rendement 63%).

On peut préparer de même d'autres 3-méthoxy-4'-benzyloxyflavones.

d) Préparation de la 4'-hydroxy-3-méthoxyflavone

On chauffe au reflux la 3-méthoxy-4-benzyloxyflavone (1 mmole) dans un mélange d'acide chlorhydrique concentré (2 ml) et d'acide acétique concentré (2 ml) pendant 3 heures. Après dilution avec de l'eau on sépare le précipité formé par filtration et on recristallise dans l'éthanol (rendement 72%) F°C = 232—233°C.

On peut préparer de même d'autres 3-méthoxyflavones.

Les composés qui sont utilisés dans la présente invention inhibent la reproduction des picorna virus, y compris le virus de l'hépatite A, les rhabdovirus et d'autres virus provoquant des maladies humaines ou animales.

On donnera ci-après dans le tableau I des résultats de l'étude in vitro de l'activité antivirale des composés utilisés dans la présente invention vis-à-vis de certains picorna virus.

L'activité antivirale a été exprimée par le facteur de réduction du titre viral par la méthode dite EPTT (end point titration technique) (c.f. Vanden Berghe et coll. Lloydia, 1978, 41, 463).

TABLEAU I

| Composés | Concentration (ug/ml) | Polio | virus Coxsackie | Rhinovirus |
|---|---|---|---|---|
| 4',5,7-Trihydroxy-3,6-diméthoxyflavone | 1 | $10^6$ | $10^5$ | $10^5$ |
| 4',5-Dihydroxy-3,6,7-triméthoxyflavone (penduletin) | 1 | $10^6$ | $10^6$ | $10^5$ |
| 5,7-Dihydroxy-3,4',6-triméthoxyflavone (santin) | 20 | $10^4$ | $10^3$ | $10^1$ |

On a par ailleurs déterminé la dose léthale 50% (DL50) la dose efficace 90% et 99% (DE90 et DE99) et l'indice thérapeutique 90 et 99 (IT90 et IT99) en utilisant les méthodes suivantes:

a) Préparation des composés:
Les composés ont été préparés à la dose 1 mg/ml dans unvolume maximal de 0,1 ml de diméthylsulfoyde et addition jusqu'à 12 ml d'un milieu de maintenance M-2. Les dilutions ont été effectuées avec le milieu de maintenance M-2.

b) Détermination de l'activité antivirale par la méthode dite "end point titration technique"
Das monocouches de cellures VERO dans des microplaques ont été infectées avec des dilutions par 10 du virus (Polio type I). Après fixation du virus aux cellules (60 minutes, 37°C) on ajoute les composés dans le milieu M-2 aux cultures infectées. Les plaques ont été incubées à 37°C pendant un temps permettant l'apparition de l'effet cytopathogène (CPE). On a déterminé l'activité antivirale à partir de cet effet. Le facteur de réduction du titre du virus est calculé pour chaque dilution par le rapport de la dilution du virus sans effet cytopathogène/dilution du virus + composé sans effet cytophathogène.
La dose efficace DE90 est la dose à laquelle 90% des virus sont inhibés.
La dose efficace DE99 est la dose à laquelle 99% des virus sont inhibés.

c) Détermination de la dose l'éthale
La DL50 est la concentration à laquelle 50% de la croissance des cellules normales est inhibée. Pour cette détermination on a utilisé $5.10^5$ cellules VERO en suspension dans 10 ml de milieu E199 additionné de 6% de sérum de veau (CS) et de gentamycine (20 microgrammes/ml). Les composés ont été ajoutés pour obtenir des concentrations finales de 200; 100; 75; 50; 25; 10; 5; 3; 2; 1; 0,1 et 0,01 microgrammes/ml.

d) Calcul de l'indice thérapeutique
IT90 et IT99 sont respectivement la valeur de la DL50 divisée par DE90 et DE99.
Les résultats sont données dans le tableau

TABLEAU II

| Composé | $DL_{50}$ | $DE_{90}$ | $IT_{90}$ | $DE_{99}$ | $IT_{99}$ |
|---|---|---|---|---|---|
| 3,4',6-triméthoxy-5,7-dihydroxyflavone | 50 | 1 | 50 | 10 | 5 |
| 3,6-diméthoxy-4',5,7-trihydroxyflavone | 20 | 0,5 | 40 | 1 | 20 |
| 3,6,7-triméthoxy-4',5-dihydroxyflavone | 100 | 0,5 | 200 | 5 | 20 |

Les compositions thérapeutiques selon l'invention peuvent donc être utilisées à la fois à titre curatif et à titre préventif.

Les compositions thérapeutiques selon l'invention peuvent être administrées à l'homme ou aux animaux par voie topique, orale ou parentérale.

Elles peuvent être sous la forme de préparations solides, semi-solides ou liquides. Comme exemples, on peut citer les comprimés, les gélules, les suppositoires, les solutions ou suspensions injectables, les pommades, les collyres huileux ou aqueux, les collutoires, les solutions nasales et otologiques, ainsi que les formes retard.

Dans ces compositions le principe actif est généralement mélangé avec un ou plusieurs excipients pharmaceutiquement acceptables habituels bien connus de l'homme de l'art.

Les compositions thérapeutiques administrables par voie topique peuvent contenir notamment de 0,1 à 5% en poids du principe actif.

Les compositions thérapeutiques administrables par voie orale ou parentérale peuvent contenir notamment de 1% à 60% en poids de principe actif.

La quantité de principe actif administré dépend évidemment du patient qui est traité, de la voie d'administration et de la sévérité de la maladie. Cependant, pour l'administration par la voie orale ou parentérale, on peut administrer environ de 100 à 1000 mg 3 à 6 fois/jour. Pour l'administration par la voie topique, on peut administrer de 0,1 à 100 microgrammes/$cm^2$ 3 à 6 fois par jour.

**Revendications pour les Etats contractants: BE CH DE FR GB IT LI LU NL SE**

1. Composition thérapeutique comprenant à titre de principe actif un composé de formule

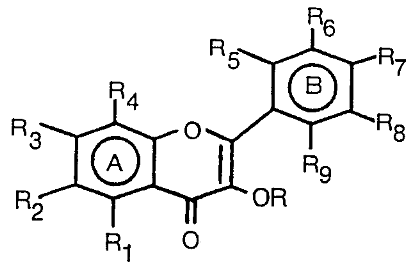

I

dans laquelle

R est un groupe alkyle en $C_1$—$C_4$,

$R_1$, $R_2$, $R_3$, $R_4$ sont choisis parmi un atome d'hydrogène, un groupe hydroxy et un groupe alkoxy en $C_1$—$C_4$, trois de ces groupes étant autres que l'hydrogène,

$R_5$ et $R_9$ sont choisis parmi un atome d'hydrogène, un groupe hydroxy et un groupe alkoxy en $C_1$—$C_4$,

$R_6$ et $R_8$ sont des atomes d'hydrogène, et

$R_7$ est un groupe hydroxy, alkoxy en $C_1$—$C_4$ ou glycosyloxy.

2. Composition selon la revendication 1, qui comprend à titre de principe actif la 5,7-dihydroxyflavone-3,4',6-triméthoxyflavone.

3. Composition selon la revendication 1, qui comprend à titre de principe actif la 4',5-dihydroxy-3,6,7-triméthoxyflavone.

4. Composition selon la revendication 1, qui comprend à titre de principe actif la 4',5,7-trihydroxy-3,6-diméthoxyflavone.

**Revendication pour les Etats contractants : AT ES GR**

Procédé de préparation d'une composition thérapeutique, caractérisée en ce qu'il consiste à mettre sous forme pharmaceutiquement acceptable un composé de formule

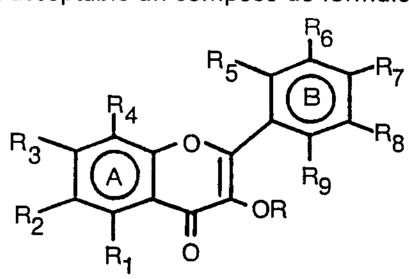

I

dans laquelle

R est un groupe alkyle en $C_1$—$C_4$ et

$R_1$, $R_2$, $R_3$, $R_4$ sont choisis parmi un atome d'hydrogène, un groupe hydroxy et un groupe alkoxy en $C_1$—$C_4$, trois de ces groupes étant autres que l'hydrogène,

$R_5$ et $R_9$ sont choisis parmi un atome d'hydrogène, un groupe hydroxy et un groupe alkoxy en $C_1$—$C_4$,

$R_6$ et $R_8$ sont des atomes d'hydrogène, et

$R_7$ est un groupe hydroxy, alcoxy en $C_1$—$C_4$ ou glycosyloxy.

**Patentansprüche für die Vertragsstaaten: BE CH DE FR GB IT LI LU NL SE**

1. Pharmazeutische Zusammensetzungen, die als Wirkstoff eine Verbindung der Formel

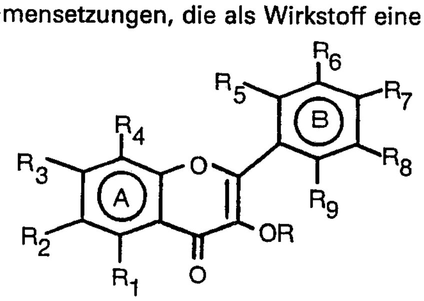

I

enthalten,

in der bedeuten:

R eine $C_{1-4}$-Alkylgruppe,

$R_1$, $R_2$, $R_3$, $R_4$ Substituenten, die ausgewählt sind unter einem Wasserstoffatom, einer Hydroxygruppe und einer $C_{1-4}$-Alkoxygruppe, wobei drei dieser Gruppen nicht Wasserstoff bedeuten,

$R_5$ und $R_9$ Substituenten, die ausgewählt sind unter einem Wasserstoffatom, einer Hydroxygruppe und einer $C_{1-4}$-Alkoxygruppe,

$R_6$ und $R_8$ Wasserstoffatome und

$R_7$ eine Hydroxy-, $C_{1-4}$-Alkoxy- oder Glycosyloxygruppe.

2. Zusammensetzungen nach Anspruch 1, die als Wirkstoff 5,7-Dihydroxy-3,4',6-trimethoxyflavon enthalten.

3. Zusammensetzungen nach Anspruch 1, die als Wirkstoff 4',5-Dihydroxy-3,6,7-trimethoxyflavon enthalten.

4. Zusammensetzungen nach Anspruch 1, die als Wirkstoff 4',5,7-Trihydroxy-3,6-dimethoxyflavon enthalten.

**Patentanspruch für die Vertragsstaaten: AT ES GR**

Verfahren zur Herstellung pharmazeutischer Zusammensetzungen, gekennzeichnet durch pharmazeutische Formulierung einer Verbindung der Formel

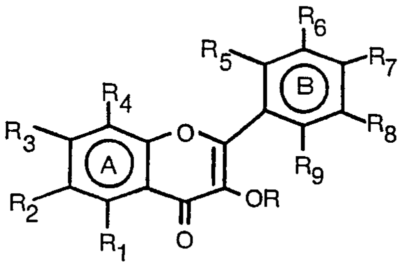

I

in der bedeuten:

R eine $C_{1-4}$-Alkylgruppe,

$R_1$, $R_2$, $R_3$, $R_4$ Substituenten, die ausgewählt sind unter einem Wasserstoffatom, einer Hydroxygruppe und einer $C_{1-4}$-Alkoxygruppe, wobei drei dieser Gruppen andere als Wasserstoff sind,

$R_5$ und $R_9$ Substituenten, die ausgewählt sind unter einem Wasserstoffatom, einer Hydroxygruppe und einer $C_{1-4}$-Alkoxygruppe,

$R_6$ und $R_8$ Wasserstoffatome und

$R_7$ eine Hydroxy-, $C_{1-4}$-Alkoxy- oder Glycosyloxygruppe.

**Claims for the Contracting States: BE CH DE FR GB IT LI LU NL SE**

1. Therapeutic composition having as the active principle a compound of formula

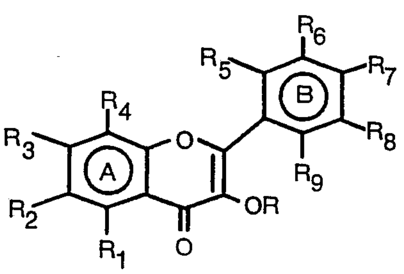

I

in which R is a $C_1$—$C_4$ alkyl group, $R_1$, $R_2$, $R_3$ and $R_4$ are chosen from among a hydrogen atom, a hydroxy group and a $C_1$—$C_4$ alkoxy group, three of these groups being other than hydrogen, $R_5$ and $R_9$ are chosen from among a hydrogen atom, a hydroxy group and a $C_1$—$C_4$ alkoxy group, $R_6$ and $R_8$ are hydrogen atoms and $R_7$ is a hydroxy, $C_1$—$C_4$ alkoxy or glycosyloxy group.

2. Composition according to claim 1 having as the active principle 5,7-dihydroxyflavone 3,4',6-trimethoxyflavone.

3. Composition according to claim 1 having as the active principle 4',5-dihydroxy-3,6,7-trimethoxyflavone.

4. Composition according to claim 1 having as the active principle 4',5,7-trihydroxy-3,6-dimethoxyflavone.

**Claim for the Contracting States: AT ES GR**

Process for the preparation of a therapeutic composition, characterized in that it consists of bringing into a pharmaceutically aceptable form a compound of formula

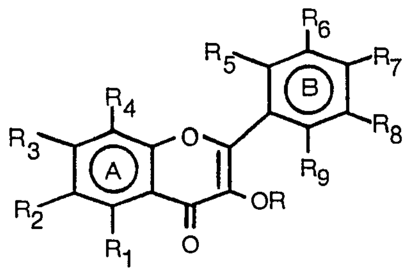

I

in which R is a $C_1$—$C_4$ alkyl group, a $R_1$, $R_2$, $R_3$ and $R_4$ are chosen from among a hydrogen atom, a hydroxy group and a $C_1$—$C_4$ alkoxy group, three of these groups being other than hydrogen, $R_5$ and $R_9$ are chosen from among a hydrogen atom, a hydroxy group and a $C_1$—$C_4$ alkoxy group, $R_6$ and $R_8$ are hydrogen atoms and $R_7$ is a hydroxy, a $C_1$—$C_4$ alkoxy or glycosyloxy group.